(19) Europäisches Patentamt
European Patent Office
Office européen des brevets

(11) **EP 2 682 155 B1**

(12) **EUROPEAN PATENT SPECIFICATION**

(45) Date of publication and mention
of the grant of the patent:
**22.07.2015 Bulletin 2015/30**

(51) Int Cl.:
***A61M 25/10*** (2013.01)

(21) Application number: **13186893.7**

(22) Date of filing: **02.12.2011**

(54) **Drug eluting balloons with ability for double treatment**

Medikamentenbeschichtete Ballons für die Zweifachbehandlung

Ballonnets d'élution de médicaments avec capacité de traitement double

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.12.2010 US 421054 P**

(43) Date of publication of application:
**08.01.2014 Bulletin 2014/02**

(62) Document number(s) of the earlier application(s) in
accordance with Art. 76 EPC:
**11799548.0 / 2 648 791**

(73) Proprietor: **Boston Scientific Scimed, Inc.
Maple Grove, MN 55311-1566 (US)**

(72) Inventors:
• **Weber, Jan**
**6228 GJ Maastricht (NL)**
• **Wetzels, Jos**
**6262 NM Banholt (NL)**

(74) Representative: **Peterreins, Frank et al
Peterreins Schley
Patent- und Rechtsanwälte
Söltlstraße 2a
81545 München (DE)**

(56) References cited:
**WO-A1-2009/096822    US-A1- 2005 037 050
US-A1- 2006 025 848    US-A1- 2009 318 848**

**Description**

**TECHNICAL FIELD**

This disclosure relates to drug-delivery balloons, as well as related medical devices and methods.

**BACKGROUND**

The body includes various passageways such as blood vessels (e.g., arteries) and body lumens. These passageways sometimes become occluded (e.g., by a tumor or plaque). To widen an occluded body vessel, balloon catheters can be used, e.g., in angioplasty.

A balloon catheter can include an inflatable and deflatable balloon carried by a long and narrow catheter body. The balloon can be initially folded around the catheter body to reduce the radial profile of the balloon catheter for easy insertion into the body.

During use, the folded balloon can be delivered to a target location in the vessel, e.g., a portion occluded by plaque, by threading the balloon catheter over a guide wire emplaced in the vessel. The balloon is then inflated, e.g., by introducing a fluid (such as a gas or a liquid) into the interior of the balloon. Inflating the balloon can radially expand the vessel so that the vessel can permit an increased rate of blood flow. After use, the balloon is typically deflated and withdrawn from the body.

U.S. Publ. No. 2005/037050 A discloses a medical device comprising a plurality of capsules, and a method of administering therapeutic agent to a patient using the same. The capsules further comprise a therapeutic agent and a multilayer polyelectrolyte shell. The medical device is adapted to apply a pressure to the capsules that is greater than or equal to the critical pressure of at least a portion of the capsules, such that therapeutic agent is released from the capsules.

U.S. Publ. No. 2006/025848 A discloses a coated medical device, such as stents and balloon catheters, for delivering a biologically active material to body tissue of a patient. The medical device has a coating layer comprising a biocompatible polymer, non-polymer material, or biologically active material disposed on its surface, and at least one structural element embedded within the coating layer. The structural elements reduce the compressibility of the coating layer. The structural element may be any shape or configuration. A biologically active material may be dispersed within the coating layer or structural elements. Methods for making such medical devices are also disclosed.

**SUMMARY**

The present invention relates to an expandable medical device as set out in claim 1. Preferred embodiments are described in dependent claims 2 to 12.

In embodiments, this disclosure relates to a medical device (e.g., a balloon catheter) that includes a sponge delivery layer including a therapeutic agent (e.g., a drug) on the outer surface of the device. The sponge delivery layer can administer the therapeutic agent upon balloon expansion, when the sponge delivery layer is compressed against a blood vessel wall. The medical device can further include a drug reservoir, which can be in the form of a drug-storage sponge layer having a different compressibility than the sponge delivery layer and including one or more therapeutic agents, and/or in the form of frangible microcapsules including one or more therapeutic agents. The sponge delivery layer can be reloaded with the one or more therapeutic agents from the drug reservoir, and can administer the therapeutic agents upon balloon re-expansion.

Embodiments of the above-mentioned medical devices can have one or more of the following features.

The first porous matrix can have a spongiform structure. The first porous matrix can include a structural element. The structural element can include silica, melamine, and/or polymethacrylate particles. The structural element can include a metal, a ceramic, a polymer, and/or a biologically active material. The structural element can include a sphere, a shell, a disk, a rod, a ridge, a strip, a wire, an oblique spheroid, a cube, or a prism.

The first layer can include a first therapeutic agent. The expandable medical device can include a second layer having a second compressibility. The second layer can include a second therapeutic agent. In some embodiments, the first layer can further include a third therapeutic agent. The first layer can be a therapeutic agent reservoir for the second layer.

The first compressibility can be at least 10 % (e.g., at least 20%, at least 30%, at least 30%, at least 40%, or at least 50%). The second compressibility can be greater than the first compressibility. The second compressibility can be less than the first compressibility.

In some embodiments, when the compressible coating is compressed, the second layer releases an amount of a second therapeutic agent from the medical device. In some embodiments, when the compressible coating is compressed, the first layer releases an amount of the first therapeutic agent into the second layer. When the compressible coating is re-compressed, the second layer can release an amount of a first therapeutic agent from the medical device.

In some embodiments, when the compressible coating is repeatedly compressed up to four times (e.g., up to three time, up to two times), the medical device releases between five micrograms and 25 micrograms (e.g., between five micrograms and 20 micrograms, between five micrograms and 15 micrograms, between five and ten micrograms) of the therapeutic agents after each of the compressions.

In some embodiments, when the compressible coating is compressed at a pressure greater than the first critical pressure of capsule rupture and less than the second critical pressure of capsule rupture, the first portion of the capsules can rupture and release the first therapeutic agent into the first layer. In some embodiments, when the compressible coating is further compressed at a pressure greater than the second critical pressure of capsule rupture, the second portion of the capsules ruptures and releases the second therapeutic agent into the first layer.

The medical device can include a balloon and/or a stent.

Embodiments and/or aspects can provide one or more of the following advantages.

The drug reservoir can increase the amount of biologically active agent that can be carried by the medical device. The multiple deliveries can result in a more uniform distribution and an increased amount of a biologically active agent to the blood vessel wall. The medical device can deliver one or more biologically active agents, which can follow a specific delivery sequence. For example, the medical device can have two or more populations of frangible microcapsules which can contain different biologically active agents. At one critical pressure, one population of microcapsules can release a biologically active agent. At a different critical pressure, a different population of microcapsules can release a different biologically active agent. In some embodiments, the structural elements can provide protection to surrounding sponge layers from possible abrasive sheer forces that can occur during introduction of a balloon into a body, during transport of the balloon, and/or during angular movements that can occur when the balloon is deployed.

The details of one or more embodiments of the disclosure are set forth in the accompanying drawings and the description below. Other features and advantages of the invention will be apparent from the description, drawings, and claims.

## DESCRIPTION OF DRAWINGS

FIG. 1A is a side-on view of a non-claimed embodiment of a balloon catheter;

FIG. 1B is an enlarged cross-sectional view of a- non-claimed embodiment of a balloon catheter wall;

FIG..2 is an enlarged cross-sectional view of a non-claimed embodiment of a balloon catheter wall when the balloon catheter is deployed;

FIG. 3A is an enlarged cross-sectional view of a- non-claimed embodiment of a balloon catheter wall;

FIG. 3B is an enlarged cross-sectional view of a non-claimed embodiment of a balloon catheter wall when the balloon catheter is deployed;

FIG. 4 is an enlarged cross-sectional view of an embodiment of a balloon catheter wall;

FIGS. 5 is an enlarged cross-sectional view of an embodiment of a balloon catheter wall; and

FIGS. 6A, 6B, and 6C are enlarged cross-sectional views of an embodiment of a balloon catheter wall when the balloon catheter is deployed.

Like reference symbols in the various drawings indicate like elements.

## DETAILED DESCRIPTION

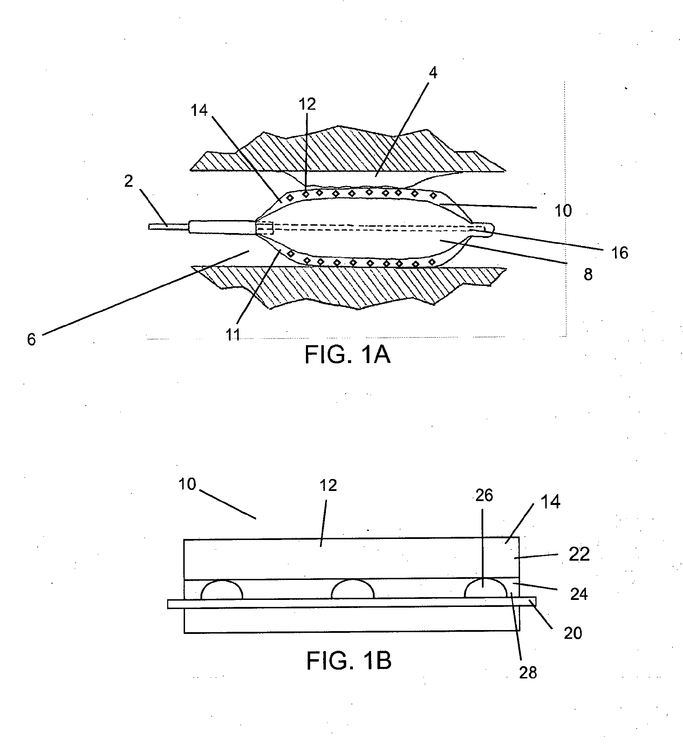

Referring to FIG. 1A, a catheter 2 can be delivered to and deployed at afflicted tissue 4 of a body lumen 6. The catheter can include an expandable portion 8 having a balloon 10 disposed about the catheter. The outer surface of balloon 10 is covered with a compressible sponge delivery layer 11 having a plurality of voids 12 therein. In some embodiments, the voids are interconnected. The sponge delivery layer 11 can include a drug 14. An inflation lumen 16 is connected to the balloon 10 to fill the balloon with inflation fluid, or pressurized gas, to expand balloon 10. A protective sheath (not shown) can be placed around the expandable portion 8 to protect the drug from inadvertent release during the insertion of the catheter in to the body lumen.

The balloon can further include a drug reservoir. In some embodiments, referring to FIG. 1B, the balloon 10 has a surface 20 that is coated by a sponge delivery layer 22 and sponge reservoir layer 24. The reservoir layer 24 can include at least one structural element 26, which can be completely surrounded, or partially surrounded, by a compressible sponge reservoir layer 24. When the structural element is partially coated, the top of the structural elements 26 can be exposed relative to sponge reservoir layer 24. Sponge delivery layer 22 can be disposed over sponge reservoir layer 24. Sponge reservoir layer 24 can operate as a drug reservoir for sponge delivery layer 22, and can contain a therapeutic agent 28, which can be the same or different therapeutic agent as therapeutic agent 14 in layer 22. In some embodiments, sponge layer 24 contains two or more therapeutic agents.

In some embodiments, instead of forming layers on a balloon, sponge delivery layer 22 and sponge reservoir layer 24 can be a uniform layer formed of the same material and having the same morphology. Structural elements 26 can be distributed throughout the sponge layers. For example, structural elements 26 can be in layer 22, layer 24, and/or partially in both layers. In some embodiments, the structural elements can protrude from layer 22, such that the structural elements can provide protection to surrounding sponge layers from possible abrasive sheer forces that can occur during introduction of a balloon into a body, during transport of the balloon, and/or during angular movements that can occur when the balloon is deployed.

In some embodiments, during deployment, a medical device, such as a balloon having layers 22 and 24, is repeatedly expanded and deflated. When first inflated at a predetermined compressive force, delivery layer 22 can be selectively compressed against a blood vessel wall to deliver an amount of a therapeutic agent 14 contained therein. The balloon can then be deflated, and re-expanded at a greater compressive force to compress delivery layer 22 together with reservoir layer 24. Upon compression, reservoir layer 24 can release a therapeutic agent 28 contained therein into layer 22. In some embodiments, therapeutic agent 28 can be drawn into layer 22 upon balloon deflation. When reinflated at a predetermined compressive force, delivery layer 22 can be selectively compressed against the blood vessel wall to deliver an amount of the therapeutic agent 28. The balloon is then deflated. In some embodiments, the inflation-deflation cycle can be repeated multiple times: the balloon can be re-inflated to compress reservoir layer 24, deflated to draw therapeutic agent 28 into layer 22, then re-inflated to compress layer 22 against the blood vessel. The multiple inflation and compression can increase an amount and distribution uniformity of a therapeutic agent that is delivered to the blood vessel wall.

In some embodiments, upon balloon deflation, an amount of drug is transported from layer 24 to layer 22 by capillary force and/or by a difference in hydrophilicity or hydrophobicity between the components of the two layers. For example, delivery layer 22 can include numerous microchannels which are squeezed and emptied upon compression and refilled upon deflation. The microchannels can be made using laser ablation. In some embodiments, the drug delivery layer can be more hydrophobic than the drug reservoir layer, and a drug can be dissolved in a hydrophobic (e.g., oily) substance (e.g., paclitaxel dissolved in camphor oil). The hydrophobic drug mixture can then be drawn into the drug delivery layer upon balloon deflation.

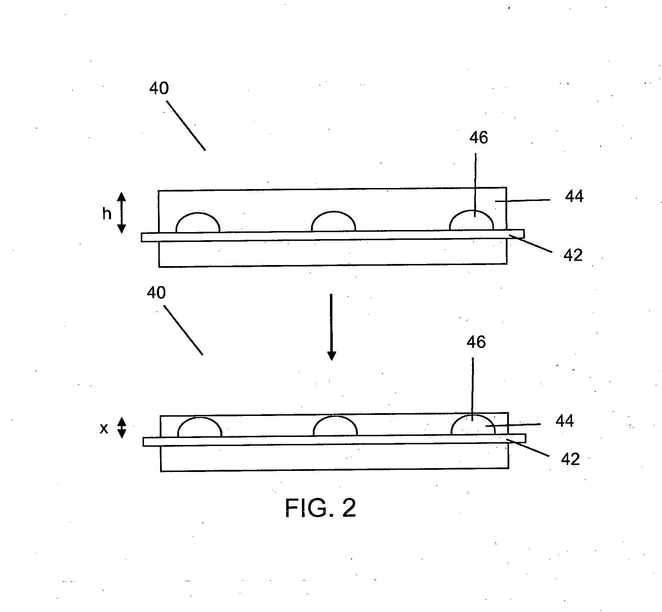

In some embodiments, sponge layers 22 and 24 include a plurality of voids and can have different compressibilities. In some embodiments, the structural elements decrease the compressibility of layer 24, i.e., the ability of the thickness of a layer to be compressed or reduced in thickness by the compressive force. To illustrate the role of the structural elements, referring to FIG. 2, an exemplary medical device 40 is shown having a surface 42 and a single sponge layer 44 including structural elements 46 disposed on the surface. Upon application of a compressive force, layer 44 is reduced from height h to x. The structural elements 46 can be formed by a material that is less compressible or harder than the materials used to form surrounding layer 44. In other words, the structural elements have a first hardness, and the material used to form layer 44, e.g., a polymer, has a second hardness that is less than the first hardness. The inclusion of the structural elements 46 in layer 44 reduces the compressibility or maximum extent to which the layer height or thickness can be reduced by a given compression force. Sponge coatings including structural elements are described, for example, in U.S. Patent Application Serial No. 10/902,747, filed July 29, 2004.

As used herein, compressibility is defined as the ability of the thickness of a layer to be compressed or reduced in height upon application of a given (e.g., predetermined) compressive force. For example, a sponge layer that decreases in thickness by 10% upon application of a given compressive force has a compressibility of 10%. In other words, a sponge layer under compression of a given force having a thickness that is 90% of the thickness of a sponge layer absent the compression has a compressibility of 10%. A compressive force refers to forces applied to a medical device in all directions. This includes but is not limited to forces experienced by the medical device upon introduction and deployment into the body lumen, which may cause a coating layer to be displaced, stripped, or compacted. Compressive forces also include forces exerted on the medical device when the device reaches its destination, which may cause a coating layer to be compacted or deformed. In addition, compressive forces can include manufacturing induced compression, such as that resulting from crimping of the device or stent on a balloon.

Moreover, the compressive force applied to the sponge layer (e.g., sponge coating) can be one that is purposely applied to the sponge layer. Since the amount of compression applied to the layer can affect the rate of biologically active material released from the layer, one can apply a certain predefined or predetermined amount of a compressive force to the layer to achieve the desired release rate. The compressive force may be applied through a balloon catheter. The thickness of a sponge layer, when a compressive force is applied to the sponge layer, can be any percentage of the thickness of the sponge layer absent the compression force. For example, the thickness when the compression is applied can be at least 10%, 20%, 30%, 40%, 50%, 60%, 70%, 80%, 85%, 90%, or 95% of the thickness of the sponge layer absent the compression force, which corresponds to a compressibility of at most 90%, 80%, 70%, 60%, 50%, 40%, 30%, 20%, 15%, 10%, or 5%, respectively. In some embodiments, the compressibility of a sponge layer is at least 5% (e.g., at least 10%, at least 15%, at least 20%, at least 30%, at least 40%, at least 50%, at least 60%, at least 70%, at least 80%, or at least 90%) and/or at most 95% (e.g., at most 90%, at most 80%, at most 70%, at most 60%, at most 50%, at most 40%, at most 30%, at most 20%, at most 15%, or at most 10%). Sponge layers (e.g., sponge delivery layer 22 and/or sponge reservoir layer 24) can be reversibly compressible, such that a sponge layer can recover all or part (e.g., 20%, 30%, 40%, 50%, 60%, 70%, 80%, 90%, or 100%) of its initial thickness once a compressive force is removed.

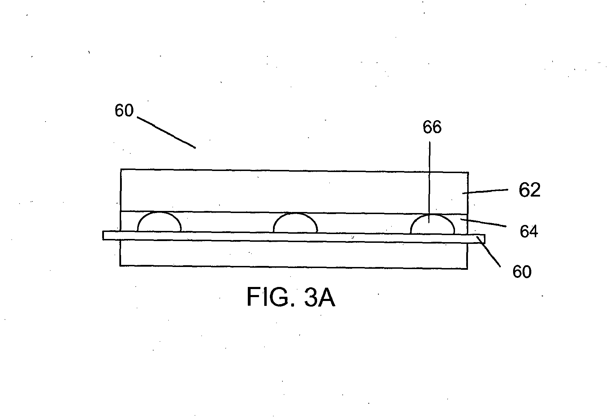

Referring back to FIG. 1B, layers 22 and 24 can be porous (e.g., have a spongiform structure). In some embodiments, sponge layers 22 and 24 can have different porous structures, such that one layer can be relatively more porous compared to the other layer. For example, referring to FIG. 3A, reservoir layer 64 can have a relatively open sponge structure that has a greater void space per volume compared to delivery layer 62. Reservoir layer 64 can contain one or more therapeutic agents.

In some embodiments, referring to FIG. 1B, layer 24 can include a porous coating material having a hardness that is greater than that of the structural elements 26 embedded or placed within layer 24. The structural elements can include a biologically active material. When layer 24 is compressed, the structural elements are squeezed and the biologically active material of the structural elements can be released into the pores of the sponge material of reservoir layer 24. The biologically active material can then be incorporated into delivery layer 22.

Prior to compression, layers 22 and 24, independently, can have thicknesses of at least 500 nanometers (e.g., at least one micrometer, at least five micrometers, at least ten micrometers, or at least 100 micrometers) and/or at most 100 micrometers (e.g., at most 50 micrometers, at most 10 micrometers, at most 5 micrometers, at most one micrometer, or at most 500 nanometers). When compressed, layers 22 and 24, independently, can have thicknesses of at least 200 nanometers (e.g., at least 500 micrometer, at least one micrometers, at least five micrometers, or at least 50 micrometers) and/or at most 50 micrometers (e.g., at most 5 micrometers, at most one micrometer, or at most 500 nanometers).

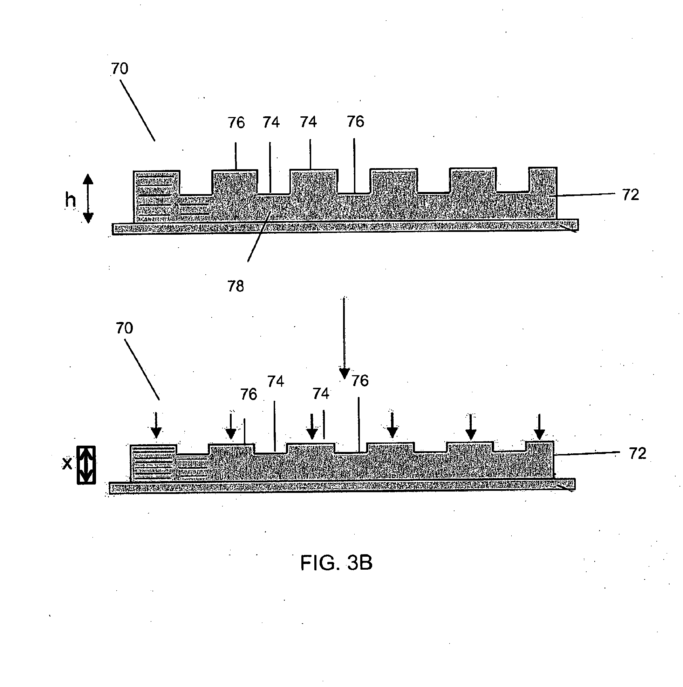

In some embodiments, instead of or in addition to having structural elements, a balloon coating can be designed with portions having different thicknesses so as to achieve controllable compressibility. For example, referring to FIG. 3B, when viewed in profile, a balloon 70 can have a balloon coating 72 having thin portions 74 and relatively thick portions 76. The thicker portions 76 can be compressed to a greater extent upon balloon inflation against a surface (e.g., a vessel wall) than portions 74, such that the compressed portions 76 perform a similar function as the structural elements shown previously in FIG. 2A. A drug 78 can be transported (e.g., by capillary forces) from portions 74 to portions 76 upon deflation of the balloon. The distance between portions 76 could range from one micrometer (e.g., from 5 micrometers, from 50 micrometers, from 100 micrometers, from 200 micrometers, or from 500 micrometers) to several hundreds of micrometers (e.g., 500 micrometers, 200 micrometers, or 100 micrometers; or to 50 micrometers, or 5 micrometers).

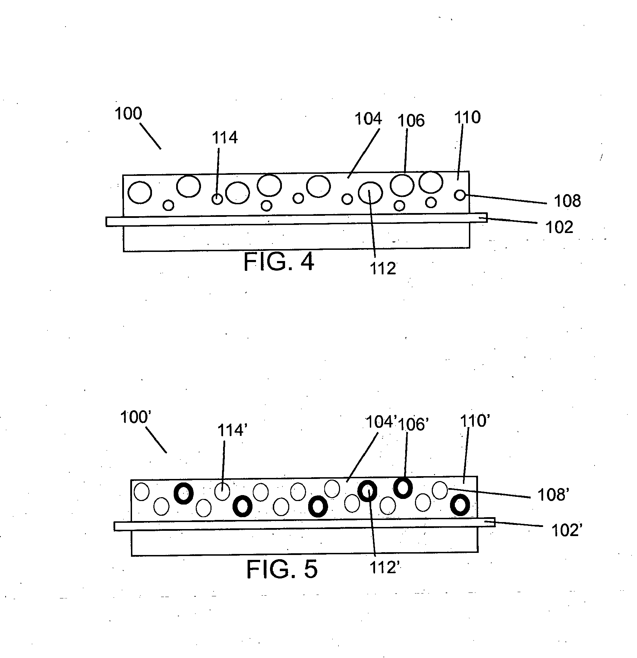

In some embodiments, the drug reservoir is in the form of frangible microcapsules. Referring to FIG. 4, the outer surface 102 of balloon 100 is covered with a sponge layer 104, which can contain therein a therapeutic agent 110. Sponge layer 104 can further include two or more populations of frangible capsules, such as capsule populations 106 and 108. Therapeutic agents 110, 112, and 114 can be the same or different. The capsules can each enclose therapeutic agents 112 and/or 114 within a multilayer polyelectrolyte shell. Polyelectrolyte capsules and methods of making thereof are described, for example, in U.S. Patent No. 7,364,585. In some embodiments, the capsules are ceramic capsules. Ceramic capsules are described, for example, in U.S. patent application serial No. 11/893,849, filed August 17, 2007.

The populations of microcapsules can have different critical pressures. In some embodiments, referring to FIG. 4, populations of capsules can have different critical pressures due to their different average diameters, such that particles having smaller diameters have a higher critical pressure. In some embodiments, referring to FIG. 5, populations of capsules (e.g., 106', 108') can have different critical pressures due to their different shell (e.g., a polyelectrolyte shell, a ceramic shell) thicknesses, such that a thicker shell affords a higher critical pressure. As used herein, critical pressure is the pressure associated with the rupture of a capsule due to deformation. In some embodiments, the critical pressure associated with spherical capsules has been reported to be predicted by the following formula:

$$\text{Critical pressure} = 4 \cdot \text{Elastic modulus} \cdot (\text{wall thickness/capsule diameter})^2$$

See C. Gao et al., "Elasticity of hollow polyelectrolyte capsules prepared by the layer-by-layer technique", European Physics Journal E 5, 21-27 (2001).

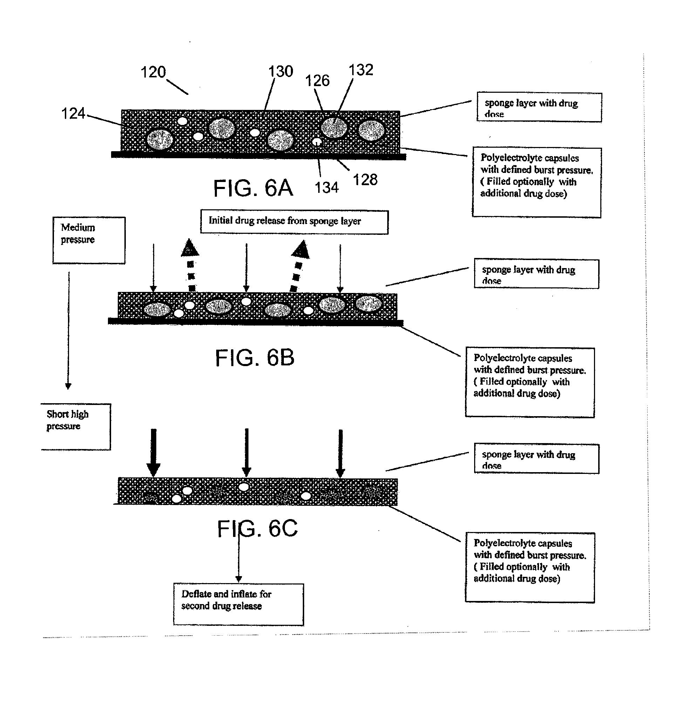

In some embodiments, referring to FIGS. 6A-6C, during deployment, a medical device (e.g., a balloon 120), is repeatedly expanded and deflated. When first inflated at a predetermined compressive force that is less than the critical pressures of capsules 126 and 128, sponge layer 124 can be selectively compressed against a blood vessel wall to deliver an amount of a therapeutic agent 130 contained therein. The balloon is then deflated, and re-expanded to compress capsules 126 at its critical pressure. Capsules 126 can burst to release therapeutic agent 132 into layer 124. In some embodiments, therapeutic agent 132 can diffuse into layer 124. In some embodiments, layer 124 expands upon balloon deflation, and therapeutic agent 132 is drawn into and redistributed within layer 124. When re-inflated, delivery layer 124 can be selectively compressed against the blood vessel wall to deliver an amount of the therapeutic agent 132. The balloon is then deflated. The balloon can be re-inflated to compress capsules 128 at its critical pressure. Capsules 128 can burst to release therapeutic agent 134 into layer 124. Therapeutic agent 134 can diffuse into layer 124. In some embodiments, layer 124 expands upon balloon deflation, and therapeutic agent 134 is drawn into and redistributed within layer 124. When re-inflated, delivery layer 124 can be selectively compressed against the blood vessel wall to deliver an amount of therapeutic agent 134. The inflation-deflation cycle can be repeated multiple times, at different compression forces, depending on the number of capsule populations. In some embodiments, balloon expansion for delivery of a therapeutic agent in layer 124 can simultaneously compress one or more populations of capsules to release a therapeutic agent contained within the capsules. The multiple inflation and compression cycle can increase the amount and distribution uniformity of therapeutic agent that is delivered to the blood vessel.

While the foregoing is directed to balloons and balloon catheters, the coated medical devices can include other devices that can be inserted and/or implanted in the body of a patient. For example, the medical device can include, but are not limited to, stents, catheters, such as balloon catheters, central venous catheters, and arterial catheters, guidewires, cannulas, cardiac pacemaker leads or lead tips, cardiac defibrillator leads or lead tipsvascular or other grafts. Medical devices are described, for example, in U.S. Patent No. 7,371,257; U.S. Patent No. 6,290,721, U.S. Patent No. 5,195,969, U.S. Patent No. 5,270,086, U.S. published patent application 2004/0044397, and U.S. Patent No. U.S. 6,287,331

The medical devices can include those that have a tubular or cylindrical-like portion. The tubular portion of the medical device need not be completely cylindrical. For instance, the cross-section of the tubular portion can be any shape, such as rectangle, a triangle, etc. Such devices include, without limitation, stents, bifurcated stents, balloon, catheters, and grafts.

In some embodiments, the medical devices can be fabricated from metallic, ceramic, or polymeric materials, or a combination thereof. Suitable metallic materials include metals and alloys based on titanium (such as nitinol, nickel titanium alloys, thermo-memory alloy materials), stainless steel, tantalum, nickel-chrome, or certain cobalt alloys including cobalt-chromium-nickel alloys such as Elgiloy® and Phynox®. Metallic materials also include clad composite filaments, such as those disclosed in WO 94/16646.

Suitable ceramic materials can include, but are not limited to, oxides, carbides, or nitrides of the transition elements such as titanium oxides, hafnium oxides, iridium oxides, chromium oxides, aluminum oxides, and zirconium oxides. Silicon based materials, such as silica, may also be used.

The polymer(s) useful for forming the medical device should be ones that are biocompatible with minimal irritation to body tissue. They can be either biostable or bioabsorbable. Suitable polymeric materials include without limitation polyurethane and its copolymers, silicone and its copolymers, ethylene vinyl-acetate, polyethylene terephthalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for medical devices include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) copolymer, polylactic acid, poly($\gamma$-caprolactone), poly($\gamma$-hydroxybutyrate), polydioxanone, poly($\gamma$-ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or cross-linking groups, e.g., RGD, in which the polymers retain their structural integrity while allowing for attachment of cells and molecules, such as proteins, nucleic acids, and the like.

The structural elements can be made of many different materials. Suitable materials include, but are not limited to, the materials from which the medical device is constructed as listed above. Also, the material of the structural elements may be porous or nonporous. Porous structural elements can be microporous, nanoporous or mesoporous. In some embodiments, structural elements can be made by crosslinking part of a coating layer (e.g., part of coating layer 22 and/or 24 in FIG. 1B) by applying either localized heat or and UV light.

In some embodiments, structural elements are fabricated from metallic, ceramic, or polymeric materials, or a combination thereof. Suitable metallic materials include metals and alloys based on titanium (such as nitinol, nickel titanium alloys, thermo-memory alloy materials), stainless steel, tantalum, nickel-chrome, or certain cobalt alloys including cobalt-chromium-nickel alloys such as Elgiloy® and Phynox®. The structural element may also include parts made from other metals such as, for example, gold, platinum, or tungsten.

The polymeric material for the structural elements may be biostable. Also, the polymeric material may be biodegradable. Suitable polymeric materials include, but are not limited to, styrene isobutylene styrene, polyetheroxides, polyvinyl alcohol, polyglycolic acid, polylactic acid, polyamides, poly-2-hydroxy-butyrate, polycaprolactone, poly(lactic-co-glycolic)acid, and Teflon. Suitable ceramic materials include, but are not limited to, oxides of the transition elements such as titanium oxides, hafnium oxides, iridium oxides, chromium oxides, and aluminum oxides. Silicon based materials may also be used.

In some embodiments, a structural element can include a porous material such as a mesoporous or nanoporous ceramic. Therapeutic agents can be introduced into the pores of structural elements made of porous material. It is also possible to optionally fill the porous structures with a biodegradable substance that would delay the release of the biologically active agent from the pores. Suitable biodegradable substances for this purpose include, but are not limited to, a polysaccharide or a heparin.

The structural elements can be fabricated from the biologically active material or any other biodegradable material such as polyelectrolyte biodegradable shells. In certain embodiments, the structural elements are biodegradable structural elements that have a hardness that is greater than that of the sponge coating layer material. These structural elements include biologically active material. Such material is released at least in part by compression of the coating layer, i.e., the release rate of the biologically active material is based at least in part on the amount of compression applied to the coating layer. When the coating layer comprising such structural elements is compressed, the biologically active material will be released. Since the structural elements comprise a biodegradable material, more drugs can be released as compared to structural elements that do not comprise a biodegradable material.

In some embodiments, at least two of the structural elements may be interconnected. The interconnected structural elements may form a lattice network of any material, such as a network of stainless steel fibers, bucky paper, or a porous ePTFE sheath. Structural elements can also be a variety of shapes such as, but not limited to, spheres, shells, discs, rods, struts, rectangles, cubics, oblique spheroids, triangles, pyramidals, tripods, or matrices, or a combination thereof.

Moreover, the structural elements can be homogeneous i.e., the structural element has the same chemical or physical properties through the entire structural elements. Also, the structural element can be multi-sectioned in which the structural element exists as sections having different chemical or physical properties. For example, a structural element can be made of a ceramic core with an overlaying electrolyte shell. The structural elements can also be multi-layered, i.e., have more than one layer. The structural elements may also be disposed evenly or unevenly in the sponge layer.

The structural elements suitable for the invention may be any size or height. Preferably, the structural element has a height that is no greater than the thickness of the sponge coating layer. For example, the structural elements may only be a percentage of the height of the coating layer. In certain embodiments the height or thickness of the structural element is at most 100%, 99%,95%, 90%,85%, 80%, 75%, 70%, 65%, 60%, 55%, 50%, 45%, 40%, 35%,30%, 25%, 20%, 15%, 10% or 5% of the thickness of the sponge coating layer containing the structural elements. Furthermore, the structural elements may vary in size.

The structural elements may be positioned in any desired pattern or distribution on the medical device. For example, when the medical device is a stent, the structural elements may be disposed on the outer surface of the stent, the inner surface of the stent, the side surfaces such as between the struts of a stent, or any combination thereof.

The structural elements may be embedded into a sponge layer using any suitable method. Preferably, the structural elements are applied to the medical device at the same time that the sponge coating layer is formed, such as by pre-mixing the structural elements with the coating composition and applying the coating composition onto the surface of the medical device to form the coating layer with the structural elements embedded therein.

The structural elements may also be applied after the sponge coating layer has been formed on the surface of the medical device. For instance, the structural elements may also be embedded into the coating layer using electrostatic forces as disclosed in copending application No. 10/335,510 to Weber, filed December 30, 2002. Another method for introducing structural elements into the coating layer is to place the structural elements into the coating layer using nano-robots or other production systems that provide micro- or nanoscale precision which are commercially available. For example, placement systems manufactured by Klocke Nanotechnik of Germany may be used. Still another method for disposing the structural elements into the coating layer is to form cavities in the coating layer of the medical device and then insert the structural elements into the cavities. The cavities may be formed by laser ablation. In this embodiment, the structural elements may comprise a porous material, and the biologically active material may be contained within the pores of the structural element.

The structural elements may be disposed on the surface of the medical device before the sponge coating composition is applied. One method of disposing the structural elements on to the surface of the medical device is to manufacture the medical device using a mold that already includes the structural elements on the surface. Another method is to weld the structural elements on to the surface of the medical device. Still another method is to etch the structural elements out of the surface of the medical device using a laser. A further method includes applying a polymer layer onto the surface of the medical device then using laser ablation to create a pattern in the first polymer. The pattern can function as the structural elements. A second polymer that is softer than the first polymer is applied over or around at least part of the pattern to form a coating layer. Additionally, an inkjet printer may be used to position the hard polymer structures prior to depositing the softer topcoating layer. Structural elements are described, for example, in U.S. Patent Application Serial No. 10/902,747, filed July 29, 2004.

In one method of forming the aforementioned sponge coating layers, a coating material composition is applied to the surface. Sponge coating compositions can be applied by any method to a surface of a medical device to form a coating layer. Examples of suitable methods include, but are not limited to, spraying such as by conventional nozzle or ultrasonic nozzle, dipping, rolling, electrostatic deposition, and a batch process such as air suspension, pancoating or ultrasonic mist spraying. Also, more than one coating method can be used to make a medical device. Coating compositions suitable for applying a sponge coating to the devices of the present invention can include a polymeric material dispersed or dissolved in a solvent suitable for the medical device, wherein upon applying the coating composition to the medical device, the solvent is removed. Such systems are commonly known to the skilled artisan.

In some embodiments, porous (e.g., microporous) polymer films can be produced by a templating technique based on the self-assembly of water droplets known as the 'breath figure' (BF) technique. The coating can be sprayed from a polymer solution containing one or more volatile solvent under conditions of high humidity onto a balloon surface. The cooling caused by solvent evaporation can include condensation of water droplets (e.g., water microspheres) onto the polymer solution surface. These water microspheres can self-assemble into hexagonally packed arrays. The droplets can create an ordered template, where the polymer can precipitate at the water interface, effectively stabilizing the droplets from coalescence. Ultimately, 3-D ordered porous films can be created.

In some embodiments, porous (e.g., microporous) polymer films on balloon surfaces can be formed using electro-spraying techniques. A polymer can be dissolved in a very volatile solvent, for example styrene-isobutylene-styrene in toluene, and the distance between a balloon surface and a spray nozzle is chosen such that a spray forms nearly dry polymer particles upon hitting of the balloon surface. The nearly dry particles can fuse together leaving an interconnected porosity. The production of a multilayer coating, such as that shown in FIG. 1B, can be formed using this technology. The very open layer 24 can be created by spraying an initial porogen (e.g., sucrose, salt) onto the balloon surface, after which a toplayer 22 can be created using an electrospray method. After drying, the porogen can be removed by elution using water. Structural elements 20 can be printed on the balloon surface before applying the porogen layer.

Various other technologies to produce microporous coatings are, for example, described in U.S. Patent No. 4,962,170, and U.S. patent application publication No. 2010/0008959.

The polymeric sponge material should be a material that is biocompatible and avoids irritation to body tissue. Preferably the polymeric materials used in the sponge coating composition of the present invention are selected from the following: polyurethanes, silicones (e.g., polysiloxanes and substituted polysiloxanes), and polyesters. Also preferable as a polymeric material are styrene-isobutylene-styrene copolymers. Other polymers which can be used include ones that can be dissolved and cured or polymerized on the medical device or polymers having relatively low melting points that can be blended with biologically active materials. Additional suitable polymers include, thermoplastic elastomers in general, polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate, copolymers of vinyl monomers, copolymers of vinyl monomers and olefins such as ethylene-methyl methacrylate copolymers, acrylonitrile-styrene copolymers, ABS (acrylonitrile-butadiene-styrene) resins, ethylenevinyl acetate copolymers, polyamides such as Nylon 66 and polycaprolactone, alkyd resins, polycarbonates, polyoxymethylenes, polyimides, polyethers, epoxy resins, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, polylactic acid-polyethylene oxide copolymers, EPDM (ethylene-propylene-diene) rubbers, fluorosilicones, polyethylene glycol, polysaccharides, phospholipids, and combinations of the foregoing.

Preferably, for medical devices which undergo mechanical challenges, e.g., expansion and contraction, polymeric sponge materials should be selected from elastomeric polymers such as silicones (e.g., polysiloxanes and substituted polysiloxanes), polyurethanes, thermoplastic elastomers, ethylene vinyl acetate copolymers, polyolefin elastomers, and EPDM rubbers. Because of the elastic nature of these polymers, the coating composition is capable of undergoing deformation under the yield point when the device is subjected to forces, stress or mechanical challenge.

Solvents used to prepare coating compositions include ones which can dissolve or suspend the polymeric material in solution. Examples of suitable solvents include, but are not limited to, tetrahydrofuran, methylethylketone, chloroform, toluene, acetone, isooctane, 1,1,1-trichloroethane, dichloromethane, isopropanol, IPA, and mixture thereof.

Therapeutic agents

The sponge coating layer may also contain one or more biological active materials. A biologically active material can also be included in the structural elements and/or in microcapsules. The term "biologically active material" encompasses therapeutic agents, such as biologically active agents, and also genetic materials and biological materials. The therapeutic agent used in embodiments of the present disclosure may be a pharmaceutically-acceptable agent such as a drug, a non-genetic therapeutic agent, a biomolecule, a small molecule, or cells. Example drugs include antiproliferative agents or anti-restenosis agents such as paclitaxel, sirolimus (rapamycin), tacrolimus, everolimus, and zotarolimus.

Exemplary non-genetic therapeutic agents include anti-thrombogenic agents such heparin, heparin derivatives, prostaglandin (including micellar prostaglandin E1), urokinase, and PPack (dextrophenylalanine proline arginine chloromethylketone); antiproliferative agents such as enoxaparin, angiopeptin, sirolimus (rapamycin), tacrolimus, everolimus, zotarolimus, monoclonal antibodies capable of blocking smooth muscle cell proliferation, hirudin, and acetylsalicylic acid; anti-inflammatory agents such as dexamethasone, rosiglitazone, prednisolone, corticosterone, budesonide, estrogen, estrodiol, sulfasalazine, acetylsalicylic acid, mycophenolic acid, and mesalamine; antineoplastic/anti-proliferative/anti-mitotic agents such as paclitaxel (e.g., paclitaxel, paclitaxel analogues, derivatives, and mixtures thereof), epothilone, cladribine, 5-fluorouracil, methotrexate, doxorubicin, daunorubicin, cyclosporine, cisplatin, vinblastine, vincristine, epothilones, endostatin, trapidil, halofuginone, and angiostatin; anti-cancer agents such as antisense inhibitors of c-myc oncogene; antimicrobial agents such as triclosan, cephalosporins, aminoglycosides, nitrofurantoin, silver ions, compounds, or salts; biofilm synthesis inhibitors such as non-steroidal anti-inflammatory agents and chelating agents such as ethylenediaminetetraacetic acid, O,O'-bis (2-aminoethyl) ethyleneglycol-N,N,N',N'-tetraacetic acid and mixtures thereof; antibiotics such as gentamycin, rifampin, minocyclin, and ciprofloxacin; antibodies including chimeric antibodies and antibody fragments; anesthetic agents such as lidocaine, bupivacaine, and ropivacaine; nitric oxide; nitric oxide (NO) donors such as linsidomine, molsidomine, L-arginine, NO-carbohydrate adducts, polymeric or oligomeric NO adducts; anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, enoxaparin, hirudin, warfarin sodium, Dicumarol, aspirin, prostaglandin inhibitors, platelet aggregation inhibitors such as cilostazol and tick antiplatelet factors; vascular cell growth promotors such as growth factors, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; inhibitors of heat shock proteins such as geldanamycin; angiotensin converting enzyme (ACE) inhibitors; beta-blockers; βAR kinase (βARK) inhibitors; phospholamban inhibitors; protein bound particle drugs such as ABRAXANE™; structural protein (e.g., collagen) cross-link breakers such as alagebrium (ALT-711); and/or any combinations and prodrugs of the above.

Exemplary biomolecules include peptides, polypeptides and proteins; oligonucleotides; nucleic acids such as double or single stranded DNA (including naked and cDNA), RNA, antisense nucleic acids such as antisense DNA and RNA, small interfering RNA (siRNA), and ribozymes; genes; carbohydrates; angiogenic factors including growth factors; cell cycle inhibitors; and anti-restenosis agents. Nucleic acids may be incorporated into delivery systems such as, for example, vectors (including viral vectors), plasmids or liposomes.

Non-limiting examples of proteins include serca-2 protein, monocyte chemoattractant proteins (MCP-1) and bone morphogenic proteins ("BMPs"), such as, for example, BMP-2, BMP-3, BMP-4, BMP-5, BMP-6 (VGR-1), BMP-7 (OP-1), BMP-8, BMP-9, BMP-10, BMP-11, BMP-12, BMP-13, BMP-14, and BMP-15. Preferred BMPs are any of BMP-2, BMP-3, BMP-4, BMP-5, BMP-6, and BMP-7. These BMPs can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNAs encoding them. Nonlimiting examples of genes include survival genes that protect against cell death, such as antiapoptotic Bcl-2 family factors and Akt kinase; serca 2 gene; and combinations thereof. Nonlimiting examples of angiogenic factors include acidic and basic fibroblast growth factors, vascular endothelial growth factor, epidermal growth factor, transforming growth factors α and β, platelet-derived endothelial growth factor, platelet-derived growth factor, tumor necrosis factor α, hepatocyte growth factor, and insulin-like growth factor. A non-limiting example of a cell cycle inhibitor is a cathespin D (CD) inhibitor. Non-limiting examples of anti-restenosis agents include p15, p16, p18, p19, p21, p27, p53, p57, Rb, nFkB and E2F decoys, thymidine kinase and combinations thereof and other agents useful for interfering with cell proliferation.

Exemplary small molecules include hormones, nucleotides, amino acids, sugars, and lipids and compounds having a molecular weight of less than 100kD.

Exemplary cells include stem cells, progenitor cells, endothelial cells, adult cardiomyocytes, and smooth muscle cells. Cells can be of human origin (autologous or allogenic) or from an animal source (xenogenic), or genetically engineered. Non-limiting examples of cells include side population (SP) cells, lineage negative (Lin-) cells including Lin-CD34-, Lin-CD34+, Lin-cKit +, mesenchymal stem cells including mesenchymal stem cells with 5-aza, cord blood cells, cardiac or other tissue-derived stem cells, whole bone marrow, bone marrow mononuclear cells, endothelial progenitor cells, skeletal myoblasts or satellite cells, muscle derived cells, go cells, endothelial cells, adult cardiomyocytes, fibroblasts, smooth muscle cells, adult cardiac fibroblasts + 5-aza, genetically modified cells, tissue engineered grafts, MyoD scar fibroblasts, pacing cells, embryonic stem cell clones, embryonic stem cells, fetal or neonatal cells, immunologically masked cells, and teratoma derived cells. Any of the therapeutic agents may be combined to the extent such combination is biologically compatible.

In some embodiments, the biologically active material may be applied with a sponge coating composition. Coating compositions suitable for applying biologically active materials to the devices of the present invention preferably include a polymeric material and a biologically active material dispersed or dissolved in a solvent which does not alter or adversely impact the therapeutic properties of the biologically active material employed. Suitable polymers and solvents include, but are not limited to, those listed above. In some embodiments, the biologically active material may be incorporated into frangible capsules, as described, for example, in U.S. Patent No. 7,364,585.

The method of the present invention has many advantages including providing an efficient, cost-effective, and relatively safe manufacturing process for applying a biologically active material to a medical device. The present method provides a medical device having a coating layer that is reasonably durable and resistant to the compressive forces applied to the coating layer during delivery and implantation of the medical device, and offers control over the release rate of a biologically active material from the coating layer.

## **Examples**

Example 1

A balloon surface (nylon 12 such as Vestamid L2101F) is printed with a spiral solid polymer line (step 1), after which a mixture of paclitaxel and porous polymer structure is electrosprayed as a sponge structure on top and in between the balloon surface and PVA line.

Poly(vinyl alcohol) (PVA) (Sigma-Aldrich) is dissolved in distilled water and dimethyl sulfoxide (DMSO) at a concentration of 4 g PVA per 1 dL water/DMSO (4/1 v/v) solution. The balloon is inflated at 2 atm. and rotated horizontally underneath the inkjet-printer while maintaining a distance of 4 mm between nozzle and balloon surface, using a metallic mandrel wire inside of the catheter lumen. Printing is conducted on a piezoelectric DOD inkjet printer (Dimatrix Materials Printer, DMP-2800, Dimatix Inc (Santa Clara, CA, USA). Jetting voltage and firing frequency is set to 25 V resp. 1 kHz. A spiral line pattern is printed on the balloon surface with a gap of 1 mm. between two adjacent lines. Rotating the balloon at a speed of 0.5 mm / second results in a line with 160~200 $\mu$m width and 1.5~2 $\mu$m height. Using similar electro-hydrodynamic spray equipment (Terronics Development Co., Indianapolis) and process as described by Henrik Hansen in US patent application publication No. 2003/0054090, entitled Method for spray-coating medical devices a porous coating, a porous SIBS coating is created on top of the balloon surface with the previously printed PVA line. A coating formulation containing 1 weight % styrene-isobutylene-styrene in 99 weight % chloroform is prepared. The formulation in the chamber of the apparatus is electrically charged and atomized using a voltage power source connected to the apparatus that is set at 12 kV and 10-15 micro amps current. The flow rate of the coating formulation at the nozzle opening is about 0.05 ml/min. The apparatus is placed above the balloon such that the distance between its nozzle opening and the balloon is about 120 mm. This distance assures the droplets to be nearly dry at landing, creating a network of adhered nearly dry droplets with interconnected space, allowing the coating to act as a sponge. The balloon is rotated underneath the nozzle whereby a grounded plate positioned underneath the balloon is exposed to the atomized droplets of the coating formulation for about 8 minutes.

Finally a paclitaxel solution is made by dissolving 40 mg/ml paclitaxel in a 90% ethanol/ 10% water solution at 40 °C. The inflated balloon with the sponge coating is dip-coated for 2 minutes in the solution and pulled out at a speed of 2 mm\second.

Example 2

A construction is made whereby a stiff fiber was embedded in a porous coating.

Similar to Example 1, a porous SIBS coating is created on top of the balloon surface, however without printing an initial PVA line. After coating an initial 10 micrometer thick porous coating, an 8 micrometer diameter stainless steel fiber (Koolon Fiber tech, China) is spiraled around the balloon with a spiral spacing of 1 mm. The fiber is held at both ends using a drop of very viscous 40% SIBS ethanol polymer solution. On top of the initial porous SIBS layer with embedded steel fiber, another 10 micrometer layer of the porous SIBS layer is sprayed. After making the assembly, the solution is dipped into a methylcyclohexane-based paclitaxel 200 nm nanocrystal dipersion (Elan) (200 nm Ptx nanocrystals stabilized with 40% by weight lecithin).

A number of embodiments of the invention have been described. Nevertheless, it will be understood that various modifications may be made without departing from the scope of the invention. Accordingly, other embodiments are within the scope of the following claims.

**Claims**

1.  An expandable medical device (120) comprising:

    a surface (102; 102'); and
    a compressible coating disposed on at least a portion of the surface (102; 102'), comprising

    (a) a first layer (104; 104; 124) comprising a first porous matrix having a compressibility of at least 10 %, and
    (b) a plurality of therapeutic agent-encapsulating capsules (106, 108; 106', 108'; 126, 128) embedded within the first layer (104; 104; 124), wherein the plurality of capsules (106, 108; 106', 108'; 126, 128) comprises a first population of capsules (106; 108'; 126) having a first critical pressure of capsule rupture and encapsulating a first therapeutic agent (112; 114'; 132), and a second population (108; 106'; 128) of capsules having a second critical pressure of capsule rupture greater than the first critical pressure of capsule rupture, and encapsulating a second therapeutic agent (114; 112'; 134),

    **characterized in that** the first therapeutic agent (112; 114'; 132) and the second therapeutic agent (114; 112'; 134) are the same therapeutic agent.

2.  The medical device of claim 1, wherein the plurality of therapeutic agent-encapsulating capsules (106, 108; 106', 108'; 126, 128) is a plurality of therapeutic agent-encapsulating polyelectrolyte capsules.

3.  The medical device of claim 2, wherein the plurality of therapeutic agent-encapsulating capsules (106, 108; 106', 108'; 126, 128) are multilayer polyelectrolyte shells.

4.  The medical device of claim 1, wherein the plurality of therapeutic agent-encapsulating capsules (106, 108; 106', 108'; 126, 128) is a plurality of therapeutic agent-encapsulating ceramic capsules.

5.  The medical device of anyone of claims 1 to 4, wherein the first layer (104; 104; 124) further comprises a third therapeutic agent (110; 110'; 130).

6.  The medical device of anyone of claims 1 to 4, wherein the first porous matrix has a spongiform structure.

7.  The medical device of anyone of claims 1 to 6, wherein the medical device is one selected of the group consisting of a stent, a balloon, a catheter or a graft.

8.  The medical device of anyone of the preceding claims, wherein the first therapeutic agent (112; 114'; 132) and the second therapeutic agent (112'; 114; 134) is a proliferative agent or an anti-restenosis agent, preferably paclitaxel, sirolimus, tacrolimus, everolimus or zotarolimus.

9.  The medical device of anyone of the preceding claims, wherein the first and second populations of capsules (106, 108; 106', 108'; 126, 128) have different average diameters.

10. The medical device of anyone of the preceding claims, wherein the first and second populations of capsules (106, 108; 106', 108'; 126, 128) have different shell thicknesses.

11. The medical device of one of the preceding claims, wherein the first porous matrix includes a polymeric material, preferably selected from the group consisting of polyurethanes, silicones and polyesters.

12. The medical device of any of the preceding claims, wherein the medical device is configured such that when the compressible coating is repeatedly compressed up to four times, the medical device (100) releases between five micrograms and 25 micrograms of the therapeutic agents (112; 112'; 114; 114'; 132; 134) after each of the compressions.

**Patentansprüche**

1.  Ein expandierbares Medizingerät (120), umfassend:

eine Oberfläche (102; 102'); und

eine komprimierbare Beschichtung, angeordnet auf mindestens einem Teil der Oberfläche (102; 102'), umfassend

(a) eine erste Schicht (104; 104'; 124), umfassend eine erste poröse Matrix mit einer Kompressibilität von mindestens 10%, und

(b) eine Mehrzahl von ein therapeutisches Mittel umkapselnden Kapseln (106, 108; 106', 108'; 126, 128), die innerhalb der ersten Schicht (104; 104'; 124) eingebettet sind, wobei die Mehrzahl an Kapseln (106, 108; 106', 108'; 126, 128) eine erste Population an Kapseln (106; 108'; 126) umfasst, die einen ersten kritischen Kapselberstdruck aufweisen und ein erstes therapeutisches Mittel (112; 114'; 132) umkapseln, und eine zweite Population an Kapseln (108; 106'; 128) umfasst, die einen zweiten kritischen Kapselberstdruck aufweisen, welcher größer als der erste kritische Kapselberstdruck ist, und ein zweites therapeutisches Mittel (114; 112'; 134) umkapseln,

**dadurch gekennzeichnet, dass** das erste therapeutische Mittel (112; 114'; 132) und das zweite therapeutische Mittel (114; 112'; 134) das selbe therapeutische Mittel ist.

2. Das Medizingerät gemäß Anspruch 1, wobei die Mehrzahl von ein therapeutisches Mittel umkapselnden Kapseln (106, 108; 106', 108'; 126, 128) eine Mehrzahl von ein therapeutisches Mittel umkapselnden Polyelektrolyt-Kapseln (106, 108; 106', 108'; 126, 128) ist.

3. Das Medizingerät gemäß Anspruch 2, wobei die Mehrzahl von ein therapeutisches Mittel umkapselnden Kapseln (106, 108; 106', 108'; 126, 128) mehrschichtige Polyelektrolyt-Hüllen sind.

4. Das Medizingerät gemäß Anspruch 1, wobei die Mehrzahl von ein therapeutisches Mittel umkapselnden Kapseln (106, 108; 106', 108'; 126, 128) eine Mehrzahl von ein therapeutisches Mittel umkapselnden keramischen Kapseln ist.

5. Das Medizingerät gemäß einem der Ansprüche 1 bis 4, wobei die erste Schicht (104; 104'; 124) weiter ein drittes therapeutisches Mittel (110; 110'; 130) umfasst.

6. Das Medizingerät gemäß einem der Ansprüche 1 bis 4, wobei die erste poröse Matrix eine spongiforme Struktur aufweist.

7. Das Medizingerät gemäß einem der Ansprüche 1 bis 6, wobei das Medizingerät gewählt ist unter einem Stent, einem Ballon, einem Katheter, oder einem Transplantat.

8. Das Medizingerät gemäß einem der vorstehenden Ansprüche, wobei das erste therapeutische Mittel (112; 114'; 132) und das zweite therapeutische Mittel (114; 112'; 134) ein proliferatives Mittel oder ein Anti-Restenosemittel, bevorzugt Paclitaxel, Sirolimus, Tacrolimus, Everolimus oder Zotarolimus, ist.

9. Das Medizingerät gemäß einem der vorstehenden Ansprüche, wobei die erste und zweite Population an Kapseln (106, 108; 106', 108'; 126, 128) unterschiedliche durchschnittliche Durchmesser aufweisen.

10. Das Medizingerät gemäß einem der vorstehenden Ansprüche, wobei die ersten und zweiten Populationen an Kapseln (106, 108; 106', 108'; 126, 128) unterschiedliche Hüllendicken aufweisen.

11. Das Medizingerät gemäß einem der vorstehenden Ansprüche, wobei die erste poröse Matrix ein Polymermaterial beinhaltet, bevorzugt gewählt ist aus der Gruppe bestehend aus Polyurethanen, Silikonen und Polyestern.

12. Das Medizingerät gemäß einem der vorstehenden Ansprüche, wobei das Medizingerät dermaßen ausgestaltet ist, so dass, wenn die komprimierbare Beschichtung wiederholt bis zu viermal komprimiert wird, das Medizingerät (100) zwischen 5 $\mu$g und 25 $\mu$g der therapeutischen Mittel (112'; 114; 114'; 132; 134) nach jeder der Kompressionen freisetzt.

**Revendications**

1. Dispositif médical expansible (120), comprenant:

une surface (102; 102'); et

un revêtement compressible qui est disposé sur au moins une partie de la surface (102; 102'), comprenant:

(a) une première couche (104; 104; 124) comprenant une première matrice poreuse présentant une aptitude à la compression d'au moins 10 %, et

(b) une pluralité de capsules d'enrobage d'agent thérapeutique (106, 108; 106', 108'; 126, 128) incorporées à l'intérieur de la première couche (104; 104; 124), dans lequel la pluralité de capsules (106, 108; 106', 108'; 126, 128) comprend une première population de capsules (106; 108'; 126) présentant une première pression critique de rupture de capsule et enrobant un premier agent thérapeutique (112; 114'; 132), et une deuxième population (108; 106'; 128) de capsules présentant une deuxième pression critique de rupture de capsule supérieure à la première pression critique de rupture de capsule, et qui enrobe un deuxième agent thérapeutique (114; 112'; 134),

**caractérisé en ce que** le premier agent thérapeutique (112; 114'; 132) et le deuxième agent thérapeutique (114; 112'; 134) sont le même agent thérapeutique.

2. Dispositif médical selon la revendication 1, dans lequel la pluralité de capsules d'enrobage d'agent thérapeutique (106, 108; 106', 108'; 126, 128) est une pluralité de capsules de polyélectrolyte d'enrobage d'agent thérapeutique.

3. Dispositif médical selon la revendication 2, dans lequel la pluralité de capsules d'enrobage d'agent thérapeutique (106, 108; 106', 108'; 126, 128) est constituée d'enveloppes de polyélectrolyte multicouches.

4. Dispositif médical selon la revendication 1, dans lequel la pluralité de capsules d'enrobage d'agent thérapeutique (106, 108; 106', 108'; 126, 128) est une pluralité de capsules de céramique d'enrobage d'agent thérapeutique.

5. Dispositif médical selon l'une quelconque des revendications 1 à 4, dans lequel la première couche (104; 104; 124) contient un troisième agent thérapeutique (110; 110'; 130).

6. Dispositif médical selon l'une quelconque des revendications 1 à 4, dans lequel la première matrice poreuse présente une structure spongiforme.

7. Dispositif médical selon l'une quelconque des revendications 1 à 6, dans lequel le dispositif médical est un dispositif du groupe comprenant un stent, un ballonnet, un cathéter ou un greffon.

8. Dispositif médical selon l'une quelconque des revendications précédentes, dans lequel le premier agent thérapeutique (112; 114'; 132) et le deuxième agent thérapeutique (112'; 114; 134) est un agent prolifératif ou un agent anti-resténose, de préférence le paclitaxel, le sirolimus, le tacrolimus, l'éverolimus ou le zotarolimus.

9. Dispositif médical selon l'une quelconque des revendications précédentes, dans lequel les première et deuxième populations de capsules (106, 108; 106', 108', 126, 128) présentent des diamètres moyens différents.

10. Dispositif médical selon l'une quelconque des revendications précédentes, dans lequel les première et deuxième populations de capsules (106, 108; 106', 108', 126, 128) présentent des épaisseurs d'enveloppe différentes.

11. Dispositif médical selon l'une quelconque des revendications précédentes, dans lequel la première matrice poreuse comprend une matière polymère, qui est de préférence sélectionnée dans le groupe comprenant des polyuréthanes, des silicones et des polyesters.

12. Dispositif médical selon l'une quelconque des revendications précédentes" dans lequel le dispositif médical est configuré de telle sorte que lorsque le revêtement compressible est comprimé de façon répétée jusqu'à quatre fois, le dispositif médical (100) libère entre 5 microgrammes et 25 microgrammes des agents thérapeutiques (112; 112'; 114; 114'; 132; 134) après chacune des compressions.

FIG. 1A

FIG. 1B

FIG. 2

FIG. 3A

FIG. 3B

FIG. 4

FIG. 5

120  130  126  132

124

| sponge layer with drug dose |

**FIG. 6A**  134  128

Polyelectrolyte capsules with defined burst pressure. ( Filled optionally with additional drug dose)

Medium pressure

Initial drug release from sponge layer

sponge layer with drug dose

**FIG. 6B**

Polyelectrolyte capsules with defined burst pressure. ( Filled optionally with additional drug dose)

Short high pressure

sponge layer with drug dose

**FIG. 6C**

Deflate and inflate for second drug release

Polyelectrolyte capsules with defined burst pressure. ( Filled optionally with additional drug dose)

# EP 2 682 155 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2005037050 A
- US 2006025848 A
- US 90274704 A
- US 7364585 B
- US 89384907 A
- US 7371257 B
- US 6290721 B
- US 5195969 A
- US 5270086 A
- US 20040044397 A
- US 6287331 B
- WO 9416646 A
- US 10335510 B, Weber
- US 4962170 A
- US 20100008959 A
- US 20030054090 A

### Non-patent literature cited in the description

- **C. GAO et al.** Elasticity of hollow polyelectrolyte capsules prepared by the layer-by-layer technique. *European Physics Journal E,* 2001, vol. 5, 21-27